# EUROPEAN PATENT SPECIFICATION

(11) **EP 2 841 943 B1**
(45) Date of publication and mention of the grant of the patent: **27.09.2023**
(21) Application number: 13723825.9
(22) Date of filing: 23.04.2013
(51) Int. Cl.: G01N 33/52, G01N 33/84

(54) **METHOD FOR CHARACTERIZING AND/OR DETERMINING SAMPLES**
VERFAHREN ZUR CHARAKTERISIERUNG UND/ODER BESTIMMUNG VON PROBEN
PROCÉDÉ POUR LA CARACTÉRISATION ET/OU LA DÉTERMINATION D'ÉCHANTILLONS

(30) Priority: 24.04.2012 FI 20120131
(43) Date of publication of application: 04.03.2015
(73) Proprietor: Kemira Oyj, 00180 Helsinki (FI)
(72) Inventor: PIHLASALO, Sari, FI-20520 Turku (FI); HÄRMÄ, Harri, FI-20520 Turku (FI)
(74) Representative: Berggren Oy
(86) International application number: PCT/FI2013/050448
(87) International publication number: WO 2013/160547

(56) References cited:
- WO-A1-99/46600
- WO-A1-99/66333
- WO-A1-03/076939
- WO-A1-03/091725
- WO-A1-2010/128203
- US-A- 4 374 120
- US-A1- 2010 151 591
- US-B1- 6 274 382

## Description

### FIELD

This invention relates to a method for characterizing and/or determining samples, particularly in the aid of lanthanide(III) ions and ligands including an aromatic structure and 2-5 chelating heteroatoms. The invention relates also to an array for characterizing and/or determining a samples utilizing the properties of lanthanide(III) chelates.

### BACKGROUND

The publications and other materials used herein to illuminate the background of the invention, and in particular, cases to provide additional details respecting the practice. Binder arrays have been used in detection of biomolecular liquids. The basis of these arrays has been the production of a library of components on surfaces or production of e.g. ion sensitive/selective surfaces. When the array is allowed to be in contact with the sample, components from the sample may interact with the array creating a fingerprint that describes the contents of the sample.

WO 2010/128203 discloses an array wherein at least two different interacting surfaces are employed for characterizing and/or determining a sample, wherein at least one of the surfaces includes a non-specific interacting material. The non-specific interacting material interacts with the sample, at least one labeling reactant, and/or combination of the sample and at least one labeling reactant. The sample and at least one labeling reactant are introduced to the interacting surfaces of the array, such as e.g. letting them in contact with the interacting surfaces of said array. The labeling reactant either as such or in combination with the sample is adapted to change at least one electromagnetically readable property of at least one interacting surface of the array.

US 4,565,670 discloses a heterogeneous method for fluorescence spectroscopic determination of a biologically active substance, later commercialized under the brand DELFIA^{®}. According to the method, a lanthanide ion is separated from EDTA-labeled immune reactant at low pH-value in solution containing a suitable detergent, a synergistic compound and a β-diketone to amplify the fluorescence after the separation.

EP 0324 323A discloses a homogenous immunochemical assay including a luminescent lanthanide(III) chelate covalently bound to a immune reactant and one or more fluorescence-modulating substances (modulators; e.g. proteins and detergents).

According to this patent application publication the modulators that do not stem from the analyte-containing sample are added to the reaction medium to avoid the fluorescence to be measured to be affected by fluorescence-affecting substances which may be present in varying amounts in biological samples. This disclosure teaches that the ligand of the chelate has to include at least 6, preferably 8 or 9 heteroatoms to be stable enough for the application disclosed.

Schäferling teaches [Angew. Chem. Int. Ed. (2012) 51, 3532] that certain luminescent lanthanide(III) chelates can be used for the development of specific sensors for oxygen, pH, hydrogen peroxide, temperature, copper ion and small organic compounds.

WO 9946600 discloses an analyte specific method including exposing a sample to a lanthanide chelate complex including an analyte specific recognition element.

WO 03/091725 is directed to a self-diagnostic test for screening for elemental mineral imbalances in a patient based on utilizing an analysis of the reaction of mineral specific reagents to a sample from a patient.

### SUMMARY

The present invention is based on the observation that characterizing and/or determining samples can be performed in solution when a certain type of ligands capable to chelate with lanthanide(III) ions are used. By using the technology of the present invention preparation of interacting surfaces such as those of WO 2010/128203 can be avoided.

Accordingly, the technology disclosed herein provides an alternative and simple method for characterizing and/or determining a sample.

According to one aspect the present technology concerns a method for characterizing and/or determining a sample including
a) providing an array comprising at least two different and separate matrices, each matrix including
   - a ligand including an aromatic structure and 2-5 chelating heteroatoms, and
   - optionally a lanthanide(III) ion,
b) introducing one or more solutions to said matrices wherein at least one of the solutions includes
   - the sample and
   - optionally a lanthanide(III) ion *in proviso* that either said matrices or at least one of the solutions include the lanthanide(III) ion,
c) allowing the one or more solutions to dissolve said matrices;
d) detecting at a predetermined time point the signal derived from the lanthanide(III) ion in solution; and
e) characterizing and/or determining the sample by comparing the signal with
   i) at least one signal derived from the lanthanide(III) ion in the presence of dissolved matrices and the one or more of the solutions in the absence of the sample; and/or
   ii) at least one signal derived from the lanthanide(III) ion in the presence of dissolved matrices and the one or more said solutions with corresponding sample; and/or
   iii) at least one signal derived from the lanthanide(III) ion in the presence of dissolved matrices and the one or more of the solutions with known sample, wherein said ligand is selected from the group consisting of a compound of formula (II), (III) and (IV) as defined in claim 1.

According to another aspect the present technology concerns an array for characterizing and/or determining a sample, including at least two different and separate matrices including a ligand including an aromatic structure and 2-5 chelating heteroatoms, and wherein said ligand is selected from a group consisting of a compound of formula (II), (III) and (IV) as defined in claim 7.

Further objects of the present technology are disclosed in dependent claims.

### BRIEF DESCRIPTION OF THE DRAWINGS

Figure 1 illustrates an exemplary method for characterizing and/or determining a sample including one (top) and two (bottom) components able to interact with the lanthanide ion. 1 = lanthanide(III) ion; 2 = chelating ligand; 4 = sample component1; 5 = sample component2.
Figure 2 illustrates a principle of an exemplary method for characterizing and/or determining a sample including one (top) and two (bottom) components able to interact with the lanthanide ion. 1 = lanthanide(III) ion, 2 = chelating ligand; 3 = modulator; 4 = sample component1; 5 = sample component2.
Figure 3 illustrates an exemplary method according the invention.
Figure 4 illustrates of characterization and/or determination of three tea brands using two different matrices (10, 11) and the present technology. Y axis = normalized luminescence signal.
Figure 5 illustrates an example on characterization and/or determination of two lake water (top, bottom) samples using the present technology. Normalized europium signal is presented as a function of modulator concentration.
Figure 6 illustrates an example on characterization and/or determination of three tap water samples in the presence of different concentrations of various modulators.
Fig 7. illustrates an example of characterization and/or determination of four river water samples using kinetic curves. X-axis = time (min).
Fig 8. illustrates an example of characterization and/or determination of seven different river water samples using 13 modulators. The data is analyzed with principle component analysis and the two first principle components are presented.
Fig 9. illustrates an example of determination of EDTA concentration on one matrix.
Fig 10. illustrates an example of characterization and/or determination three tea samples using different matrices. The matrices were predissolved and EuCl₃ added together with the sample.
Fig 11. illustrates an example of characterization and/or determination of three household water samples. The matrix EuCl₃:NTA:TOPO was predissolved (60). The matrix NTA:TOPO was predissolved and EuCl₃ was predried (61).
Figure 12 illustrates an example of characterization and/or determination of water samples containing different concentration of Fe³⁺. The matrix TbCl₃:dipicolinic acid and TbCl₃: 2,3-dihydroxynaphtalene were predried in a well. Sample only was added.
Figure 13 illustrates an example of different ligands. The matrix TbCl₃ and ligand was predried and water was added.
Figure 14 illustrates an example of different ligands. The matrix TbCl₃ and ligand was predried and water was added.
Figure 15 illustrates an example of measuring Fe³⁺. The matrix TbCl₃ and ligand was predried and water was added.

### DETAILED DESCRIPTION OF THE INVENTION

As defined herein the term "matrix" refers to a medium including at least one ligand including 2-5 chelating heteroatoms able to chelate a lanthanide(III) ion and an aromatic moiety able to absorb excitation energy. Accordingly, the simplest matrix of the present technology is the ligand as such. However, in order to reduce the simplest matrix into practice it is essential that the lanthanide(III) ion is introduced into the matrix together or before the introduction of the sample. The matrix may also include other components such as the lanthanide(III) ion, detergents, buffer components, solvents and synergistic agents.

As defined herein the term "dissolved matrix" refers to a matrix that is at least partially dissolved by introduction of the sample solution and/or one or more solutions. The signal derived from the lanthanide(III) ion is measured from solution.

As defined herein the term "array" refers to two or more matrices suitable for determining and/or characterizing a sample, wherein each matrix includes an aromatic ligand including 2-5 chelating heteroatoms able to chelate a lanthanide(III) ion; *i.e* the ligand is able to form a luminescent lanthanide(III) chelate in the presence of a lanthanide(III) ion. If the array does not include the lanthanide ion it is essential that the lanthanide(III) ion is introduced together with the sample or in a separate addition step.

As defined herein the term "fingerprint" refers to results obtained through the detection, i.e. measurement of the sample in the presence of plurality of different matrices including one or more lanthanide ions and one or more ligands. If an embodiment of the present technology involves the use of a plurality of identical matrices and the signal derived from the lanthanide ion of more than one of these are detected, the fingerprint can include all the results of the identical matrices or alternatively only a representative value, e.g. average, median, mode of the measurements of identical lanthanide(III) chelates or any combination thereof [Bartz A. E. (1999), Basic Statistical Concepts. 4th ed., Upper Saddle River, NJ: Prentice Hall]. A fingerprint can further refer to a profile of measured luminescent intensities, lifetimes subjected to numerical processing with an appropriate algorithm and in many preferred alternatives measured luminescent intensities of the chelates of the array are subjected to numerical processing by an appropriate algorithm before comparison with fingerprints of corresponding arrays without a sample and/or known and/or corresponding samples.

As defined therein the term "corresponding sample" refers to a sample that is believed to be virtually identical, highly similar or at least reasonably similar to those characterized and/or determined. This belief of similarity can be due to e.g. origin, i.e. relating to the same or a corresponding process or product, or classification.

As defined therein the term "known sample" refers to any sample which composition is known in detail or is fully characterized.

The lanthanide chelate according to the present technology must include a ligand including an aromatic structure that is able to absorb excitation energy. For an efficient energy transfer the aromatic structure may include heteroatoms such as nitrogen (e.g. pyridine-N), or oxygen (e.g. furan-O) or sulfur (e.g. thiophen-S). The ligand must consists (taking the first mentioned chelating heteroatoms into account) 2-5, preferably 2 or 3 heteroatoms that are able to chelate the lanthanide ion. The heteroatoms may be selected from oxygen (e.g. carboxyl, sulfonate, phosphate, phosphonate, ether, -N=O, C=O), nitrogen (e.g. primary, secondary or tertiary amine or amide), phosphorus (e.g. phosphine or phosphine oxide) and sulfur (e.g. C=S).

Lanthanide chelates containing six or more chelating heteroatoms are too stable to be used according to the present technology. For example, the ligand 1 shown below is able to form a seven-dentate chelate with a lanthanide ion (the arrows indicate the chelating sites), is not suitable for the present technology. Ligands 2 and 3, in turn, form three- and two-dentate chelates with lanthanide ion, respectively, and thus are suitable for the present technology.

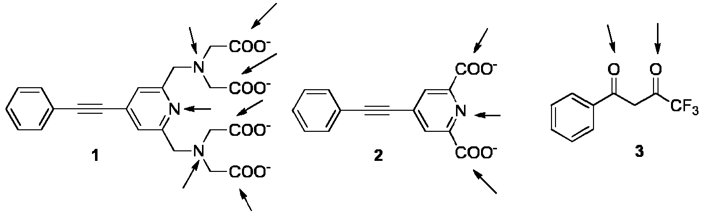

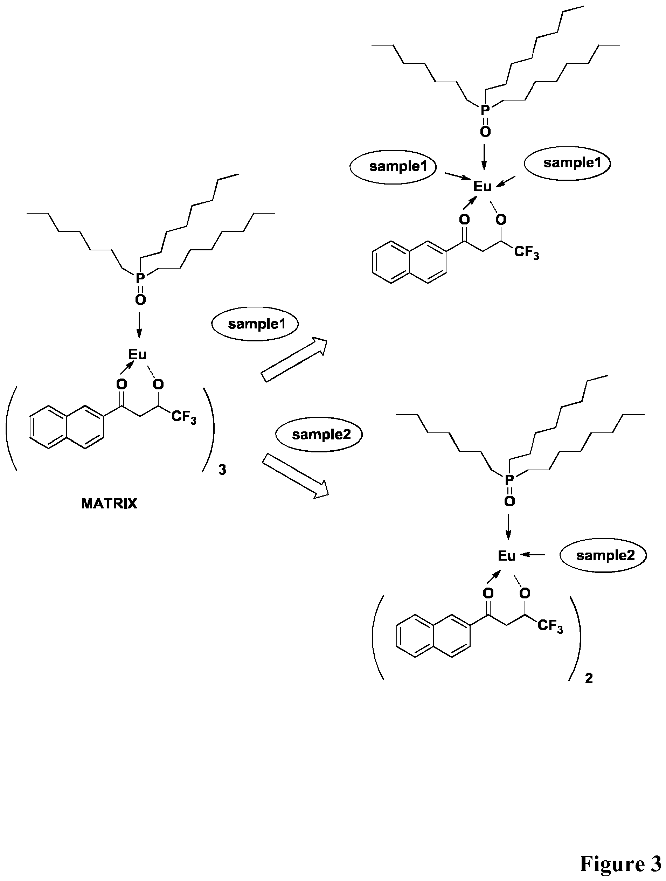

It is known in the art that a lanthanide(III) ion is able to form chelates wherein 1-4 two-dentate ligands such as **3** and up to 3 three-dentate ligands such as **2** are coordinated to a single lanthanide ion. These types of chelate structures are suitable for the present technology. An example of such a chelate is disclosed in Fig 3 wherein the matrix consists of europium, three ligands (NTA) and one modulator (TOPO).

It is known that β-diketones exhibit keto-enol tautomerism. Accordingly, it is obvious that the present disclosure includes both tautomers although only keto forms are generally presented in figures and formulas.

It is obvious for a person skilled in art that the structure of the luminescent lanthanide chelate used in the present technology varies upon addition of sample solution and other components capable to coordinate with the lanthanide ion and/or interact with each other. Furthermore, the structure of the chelate may be different at different time points. The signal observed in the present technology is derived from a lanthanide ion in the presence of one or more components with or without the sample to be determined and/or characterized.

According to the present technology the interaction of the sample with the ligand, lanthanide(III) ion, chelate and other components is non-specific. The term non-specific interaction means that the selectivity of the binding or other interaction is not predetermined. The ligand used in the method according to the present technology does not include sample specific recognition elements, such as boronates, germanates or arsenates.

According to an aspect, the present technology includes a method for characterizing and/or determining a sample, including
a) providing a matrix including
   - a ligand including an aromatic structure and 2-5 chelating heteroatoms, and
   - optionally a lanthanide(III) ion,
b) introducing one or more solutions to the matrix wherein at least one of the solutions includes
   - the sample and
   - optionally a lanthanide(III) ion;
      *in proviso* that either the matrix or at least one of the solutions include a lanthanide(III) ion;
c) allowing the one or more solutions to dissolve the matrix;
d) detecting at a predetermined time point a signal derived from the dissolved lanthanide(III) ion in solution; and
e) characterizing and/or determining the sample by comparing the signal with
   i) at least one signal derived from the dissolved lanthanide(III) ion in the presence of the dissolved matrix and the one or more solutions in the absence of the sample; and/or
   ii) at least one signal derived from the lanthanide(III) ion in the presence of the dissolved matrix and one or more the solutions with corresponding sample; and/or
   iii) at least one signal derived from the lanthanide(III) ion in the presence of the dissolved matrix and the one or more solutions with known sample.

According to an exemplary embodiment, the method includes diluting the sample with a known amount of aqueous buffer, introducing the sample solution to the matrix including the chelating ligand and the lanthanide(III) ion allowing the sample solution to dissolve the matrix, and detecting at a predetermined time point the signal derived from the lanthanide(III) ion in solution.

This embodiment further includes detecting the signal of the lanthanide(III) ion in the absence of the sample. Accordingly, a known amount of aqueous buffer is introduced to the matrix including the chelating ligand and the lanthanide(III) ion, allowing the buffer solution to dissolve the matrix, and detecting at a predetermined time point the signal derived from the lanthanide(III) ion in solution in the absence of the sample.

The sample is then characterized and/or determined by comparing the signal derived from the lanthanide(III) ion in the presence of the sample and absence of the sample.

According to another embodiment the signal of the lanthanide(III) ion in the presence of known sample is obtained by dissolving the known sample to a known amount of same aqueous buffer as the sample to be characterized and/or determined, introducing the solution including the known sample to the matrix, allowing the matrix to dissolve to the solution, and detecting at a predetermined time point the signal in solution. The sample is then characterized and/or determined by comparing the signal derived from the lanthanide(III) ion in the presence of the sample to be analyzed and the known sample.

According to another embodiment the signal of the lanthanide(III) ion in the presence of corresponding sample is obtained by dissolving the corresponding sample to a known amount of same aqueous buffer as the sample to be characterized and/or determined, introducing the solution of the corresponding sample to the matrix, allowing the matrix to dissolve to the solution, and detecting the signal derived from the lanthanide(III) ion at a predetermined time point in solution. The sample is then characterized and/or determined by comparing the signal derived from the lanthanide(III) ion in the presence of the sample to be analyzed and the signal derived from the lanthanide(III) ion in the presence of the corresponding sample.

According to an embodiment the sample is determined and/or characterized by comparing the signal derived from the lanthanide(III) ion in the presence of the sample to be analyzed to one or more of the corresponding signal of the lanthanide(III) ion without a sample, in the corresponding signal of the lanthanide(III) ion in the presence of corresponding sample and/or the corresponding signal of the lanthanide(III) ion in the presence known sample.

When the matrix does not include the lanthanide(III) ion, it is introduced separately or together with the sample solution, solution including the known sample, solution including the corresponding sample, and/or a solution without sample.

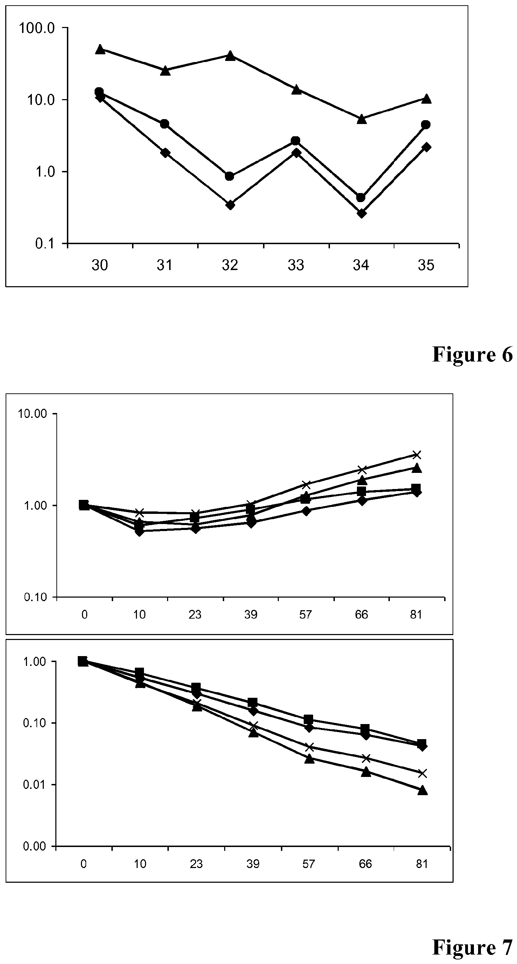

According to another embodiment the sample, matrix without a sample, the corresponding sample and/or the known sample is determined at more than one predetermined time point in solution. According to this embodiment the sample can be characterized and/or determined based on the signal derived from the lanthanide(III) ion as a function of time. An exemplary embodiment is shown in figure 7 wherein four different river water samples are determined at different time points.

According to another embodiment, the sample is characterized and/ or determined based on signal intensity and/or life time of the lanthanide signal.

The lanthanide ion of the present technology is selected from europium, terbium, samarium and dysprosium. The preferable lanthanide ion is europium(III). Another preferable lanthanide is terbium.

According to an aspect the ligand is a β-diketone of formula (I) 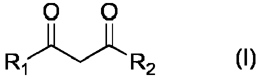 wherein R₁ is an aryl, optionally mono-or multi-substituted, and R₂ is a straight or branched alkyl chain with 1 to 9 carbon atoms substituted with four or more fluorine atoms optionally mono-or multi-substituted with other substituents than fluorine.

According to a preferable aspect R₁ is selected from the group consisting of phenyl, 9H-fluoren-2-yl, 1- naphthyl, 2-naphtyl, 2-phenanthrolyl, 3-phenanthrolyl, 4-phenanthrolyl, 5-phenanthrolyl, 2-furyl, 3-furyl, 2-benzofuryl, 3-benzofuryl, 2-thienyl, 3-thienyl, 2-pyridyl, 3-pyridyl, 4-pyridyl, 2-thiazolyl, 4-thiazolyl, 5-thiazolyl, 2-benzothiazolyl, 2-benzo [b] thienyl, 3-benzo [b] thienyl, 2-pyrimidyl, 4-pyrimidyl and 5-pyrimidyl.

When R₁ is mono-or multi-substituted each substituent is preferably independently selected from the group consisting of straight or branched alkyl, alkoxy, aryl, aroyl, aryloxy, nitro, amino, cyano, hydroxy, carboxy, chloro, bromo, fluoro and acyl. According to a preferable aspect the alkyl chain R₂ is substituted with 3 to 9 fluorine atoms. Exemplary β-diketones according to present technology are β-diketones of 4,4,4-trifluoro-1-(2-naftyl)-1,3-butanedione (NTA), 2-thienyl-trifluorooacetone (TTA) , 4,4, 5,5, 5-pentafluoro-1-aryl-1, 3-pentanedione or 4,4, 5,5, 6,6, 6-heptafluoro-1- aryl-1,3-hexanedione, 1- (2-benzofuryl)-4, 4,5, 5,5-pentafluoro- 1,3-pentanedione, 1- (2-benzofuryl)-4, 4,5, 5,6, 6, 6-heptafluoro-1, 3-hexanedione, 1- (2-benzo [b] thienyl) -4, 4,5, 5, 5-pentafluoro-1, 3-pentanedione and 1-(2-benzo [b] thienyl) -4, 4,5, 5,6, 6,6-heptafluoro-1, 3-hexanedione.

According to one preferable aspect the ligand is selected from TTA and NTA.

According to another embodiment the ligand is a compound of formula (II) 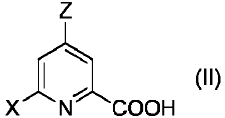 wherein Z is aryl, and X is selected from COOH and CH₂N(CH₂COOH)₂. According to a preferable embodiment the aryl is selected from a group consisting of phenyl, phenylethynyl, napthyl, biphenyl, furan, thiophene, pyridine, pyrazole, imidazole, isothiazole, oxazole, dialkoxyphenyl, and trialkoxyphenyl, preferably trimethoxyphenyl.

According to another embodiment the ligand includes only an aromatic structure that includes two or three chelating heteroatoms. An exemplary ligand according to this embodiment is 1,10-phenantroline (phen).

According to another embodiment the ligand includes two acidic chelating hydroxyl groups. Exemplary ligand according to this embodiment is 2,3-dihydroxynapthalene. According to another embodiment the ligand is a compound of formula (III) 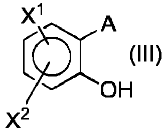 wherein A is OH or COOH, and X¹ and X² are independently selected from halogen, H, and SO₃H. Preferable compounds of formula (III) are fluorosalicylic acid, salicylic acid, and 4,5-dihydrobenzene-1,3-disulfonic acid.

According to another embodiment the ligand is a compound of formula (IV) 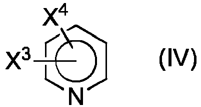 wherein X³ is selected from H, COOH and CONH₂, and X⁴ is selected from COOH and CONH₂. Preferable compounds of formula (IV) are picolinic acid, dipicolinic acid, nicotinic acid and nicotinamide

According to one embodiment the ligand is selected from the group consisting of dipicolinic acid, picolinic acid, nicotinic acid, nicotinamide, fluorosalisylic acid, 2,3-dihydronaphtalene, salicylic acid, cytosine, chelidamic acid, 4,5-dihydrobenzene-1,3-disulfonic acid, oxilinic acid, ciprofloxacin, 7-amino-1,3-naphhalenesulfonic acid, pyridinedicarboxylic acid, nalidixic acid, and 4-hydroquinoline-2-carboxylic acid.

The matrix may include one or more ligands. An exemplary matrix includes NTA and TTA. Also the molar ratio of the ligands may vary from matrix to matrix and within one matrix.

According to a preferable embodiment the matrix and/or the one or more of the solutions further includes one or more modulators preferably selected from the group consisting of a synergistic ligand, detergent, protein, peptide, aminocarboxylic acid, carboxylic acid, buffer components and carbohydrate. The function of the modulator in the present technology is preferably to enhance the signal of the lanthanide chelate and/or assist the formation of individual signal or fingerprint of the sample to be charactrerized and/or determined.

According to one embodiment the modulator competes with the sample and/or the chelating ligands on the coordination sphere of the lanthanide ion of the chelate and thus modulates the signal derived from the lanthanide(III) ion. The modulator may also enhance the signal e.g. by limiting signal quenching of water molecules.

According to another embodiment the sample and/or modulator binds to or interacts with the chelating ligand and thus modulates the signal derived from the lanthanide(III) ion.

According to one embodiment one or more of the modulators is a detergent. The detergent is preferably selected from an alkyl aryl polyether alcohol, a zwitterionic compound, and a quaternary ammonium compound. Exemplary detergents are 4-(1,1,3,3-tetramethylbutyl)phenylpolyethyl glycol (Triton X-100), sodium docecyl sulfate (SDS), polyethyleneglycol sorbinan monolaurate (Tween-20), dimethyldodecylphosphine oxide (Apo-12), 3- [(3-cholamidopropyl) dimethylammonio]-1-propanesulfonate and cetyltrimethylammonium bromide (CTAB).

According to another embodiment one or more of the modulators is a synergistic ligand. The synergistic ligand is preferably a Lewis base. A group of such bases consists of N-heterocyclic compounds such as o-phenantroline and of another group consisting of phosphine oxides and phosphines. Exemplary synergistic ligand are trioctylphosphine (TOPO), triphenylphosphine oxide (TPPO), dimethyldecylphosphine oxide (DMDPO), and tributylphosphine oxide (TBPO) and 1,10-phenantroline (phen). The modulators including an aromatic moiety may also act as ligands.

The aminocarboxylic acid is preferably EDTA and DTPA that are known to coordinate metal ions. Exemplary carboxylic acids are citric acid, acetic acid, formic acid, propionic acid and lactic acid.

According to an embodiment the matrix is predried. In the method according to the present technology, the predried matrix is dissolved upon addition of the sample solution, and the detection is performed at predetermined time point in solution. The dissolution of the matrix can be assisted e.g. by warming, stirring and/or using ultrasound. Since some of the matrix components may have limited water solubility, they can be predissolved in one or more suitable solvents. Exemplary solvents are DMSO, DMF, water, ethanol, methanol and acetone. A preferable solvent is DMSO. It is also possible that some of the matrix components are predried and some on the matrix components are predissolved into a solvent such as DMSO. According to this embodiment the matrix includes two phases: a liquid phase and a solid phase preferably as two spots at close proximity: the matrix phases will be combined upon addition of the sample solution. It is also possible that all matrix components are in solution. According to this embodiment the term dissolving is understood that two or more solutions are mixed.

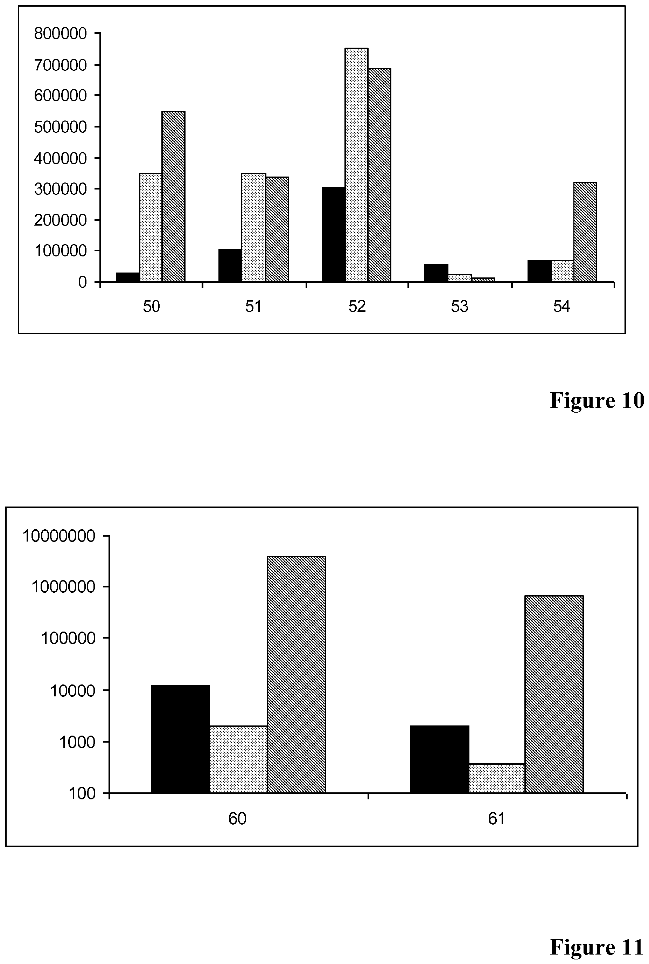

An exemplary matrix according to the present technology includes NTA and TOPO predissolved in DMSO and predried europium chloride (Figure 11; 61).

Another exemplary matrix includes NTA and TOPO in DMSO. According to this embodiment the lanthanide(III) ion is added to the matrix, e.g. as an aqueous solution of europium(III) chloride (Figure 10).

The method according to the present technology can be performed by using laboratory devices know in art. Exemplary device are microtiter plates, fluidic devices and test tubes.

The method according to the present technology can be performed in aqueous solution such as in aqueous buffer(s), it the mixture of an aqueous solution and organic solvent(s), and in organic solvent(s). It is obvious for a person skilled in art that the suitability of solvent or solvent system for the method is dependent on the nature of the reagents and reactants used.

According to another embodiment of the present technology an array of at least two different matrices is employed for characterizing and/or determining a sample. It should be understood that in an array, the at least two matrices are not mixed but used separately in the method. The separate matrices may be e.g. on microtiter plate wells, a fluidic device containing separate reaction wells or separate test tubes.

According to one embodiment the sample as a solution is introduced to the matrices and the matrices are allowed to at least partially dissolve. The matrices include a ligand including an aromatic structure and 2-5 chelating heteroatoms that are able to chelate a lanthanide(III) ion. Either the matrices or the sample solution must include the lanthanide(III) ion. The sample as such or in combination with other components present in the matrix (i.e. modulators) changes the signal derived from the lanthanide(III) ion.

According to another embodiment a concentration of a sample can be measured.

In order to obtain the fingerprint of the sample the signal derived from the lanthanide(III) ion of at least two different matrices of the array is detected at a predetermined time point in solution. The characterizing and/or determining the sample is carried out by comparing the fingerprint of the sample with at least one fingerprint of at least one corresponding sample, at least one fingerprint of an array obtained without a sample, and/or at least one fingerprint of known samples.

The signal derived from the lanthanide(III) ion may be used in revealing the fingerprint of an unknown sample by comparing the fingerprint of the signal derived from the lanthanide(III) ion in presence and/or absence of the sample. In comparative studies the fingerprint of the array is compared in presence of samples A, B, C etc. The comparisons of the acquired fingerprints against a library or comparison sets are made by known methods from bioinformatics and data mining. In many embodiments of the present invention fingerprints processed using appropriate algorithms are compared rather than observing the fingerprints of samples as such. Fingerprints and fingerprints processed using appropriate algorithms of the sample can be compared with fingerprints or fingerprints processed, respectively, using appropriate algorithms of:
- a sample from the same process taken at an earlier time point,
- a sample taken before and/or after adding a substance to the process under study,
- a fingerprint of the array in absence of the sample,
- a fingerprint of the array in presence of a different concentration of the sample,
- a library of fingerprints from known samples,
- a library of fingerprints related to known anomalities observed at the time points of the library sample,
- an algorithm trained with fingerprints of known samples,
- an algorithm trained with fingerprints from samples related to known anomalities observed at the time point of the sampling, and or
- a fingerprint or set of fingerprints recorded as a function of time.

These algorithms [see e.g. www.dtreg.com and Romesburg C, Cluster Analysis for Researchers, Lulu Press 2004] may e.g. be based on:
- neural networks,
- independent/principal component analysis,
- discriminant analysis,
- other clustering algorithms, and
- generic expression programming.

In an exemplary embodiment of the present technology the assay is performed as follows:
1) A sample is diluted in a buffer solution.
2) The sample and the buffer solution is allowed to contact two or more matrices including a chelating ligand, a lanthanide(III) ion and modulators.
3) The matrices are allowed to dissolve to the sample solution.
4) The arrays are read. Reading of results may be carried out with e.g. a luminescence plate reader, a dedicated luminescence array reader, a flow luminometric device, and/or an automated imaging device.
5) The results are interpreted by an appropriate method. The method may involve comparison to a sample from the same process taken at an earlier time point, comparison to a sample taken before and/or after adding a particular substance to the process under study, comparison to a fingerprint of the array in absence of the sample, comparison to a fingerprint of the array in presence of a different concentration of the sample, comparison to a library of fingerprints from known samples, and/or comparison to a library of fingerprints related to known anomalities observed at the time points of the library sample The method may utilize e.g. an algorithm sensitive in differentiation of multidimensional signals, an algorithm trained with fingerprints of known library samples, an algorithm trained with fingerprints from known library samples related to known anomalities observed at the time point of the sampling, and/or comparison to results from the same sample as a function of time after the addition of reaction components. Algorithms of the method may be based on e.g. neural networks, independent component analysis, discriminant analysis and other clustering algorithms and generic expression programming.

It is clear for a person skilled in the art that the presented protocol and the order of the addition of the reaction components may vary depending e.g. on the application, sample, used reaction and used analysis methods.

According to another embodiment the present technology includes a computer program including software modules for determining information indicative for the sample, in order to evaluate the signal derived from the lanthanide(III) ion under various conditions to obtain fingerprint of the sample; i.e. to characterize and/or determine the sample. The software modules can be e.g. subroutines of functions implemented with a suitable programming language and with a complier suitable for the programming language and the programmable processor.

A computer program product according to an exemplifying aspect of the present technology includes a computer readable medium e.g. a compact disc, encoded with a computer program according to an aspect of the present technology.

A signal according to the exemplary aspect is encoded to carry information defining a computer program according the embodiment.

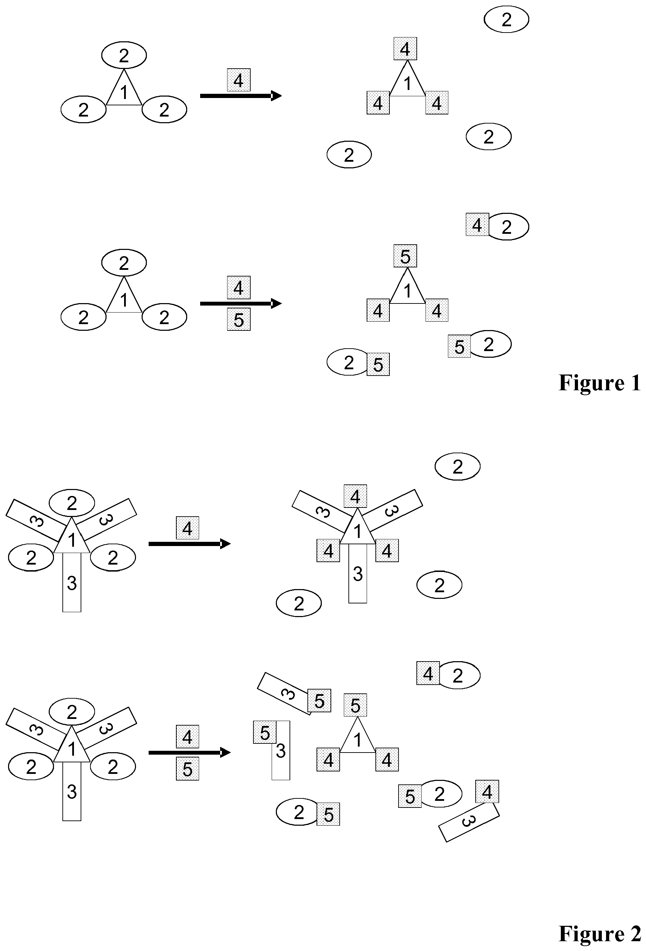

Figure 1 illustrates (top) an exemplary embodiment of the present technology. The matrix consists a lanthanide(III) chelate including a lanthanide(III) ion 1 and three chelating ligands 2. The sample includes one component 4 that is able to bind the lanthanide(III) ion. According to this embodiment the component 4 binds more strongly with the lanthanide ion than the chelating ligands. The signal observed after addition of the sample solution changes and is dependent on the nature of the sample component 4.

Figure 1 illustrates (bottom) another embodiment of the present technology. The matrix consists a lanthanide(III) chelate including a lanthanide(III) ion 1 and three chelating ligands 2. The sample solution includes two components: 4 and 5. Both components are able to coordinate the lanthanide ion and compete with chelating ligand 2. Components 4 and 5 are capable to bind the ligand 2 also. The signal observed is dependent on the nature of the components 4 and 5.

Figure 2 (top) illustrates another embodiments of the present technology. The matrix consists of a lanthanide(III) ion 1, three ligands 2 and three modulators 3. The sample solution includes one component that is able to compete with the ligand 2 on the coordination sphere of the lanthanide(III) ion. The signal derived from the lanthanide(III) ion is dependent on the sample component.

Figure 2 (bottom) illustrates another embodiments of the present technology. The matrix consists of a lanthanide(III) ion 1, three ligands 2 and three modulators 3. The sample solution includes two component that are able to compete with the ligand 2 on the coordination sphere of the lanthanide(III) ion. The components are able to bind with the ligands and the modulator also. The signal derived from the lanthanide(III) ion is dependent on the nature and the amount of sample components.

Figure 3 illustrates a further embodiment of the present technology. The matrix includes a lanthanide chelate including a europium ion, three chelating ligands (NTA) and a modulator (TOPO). Sample component 1 is able to remove major amount the chelating NTA from the coordinating sphere of Eu³⁺ giving rise to significant decrease of signal. Sample component 2 is able to remove only minor amount of NTA from the coordinating sphere of europium(III) giving rise to moderate of signal.

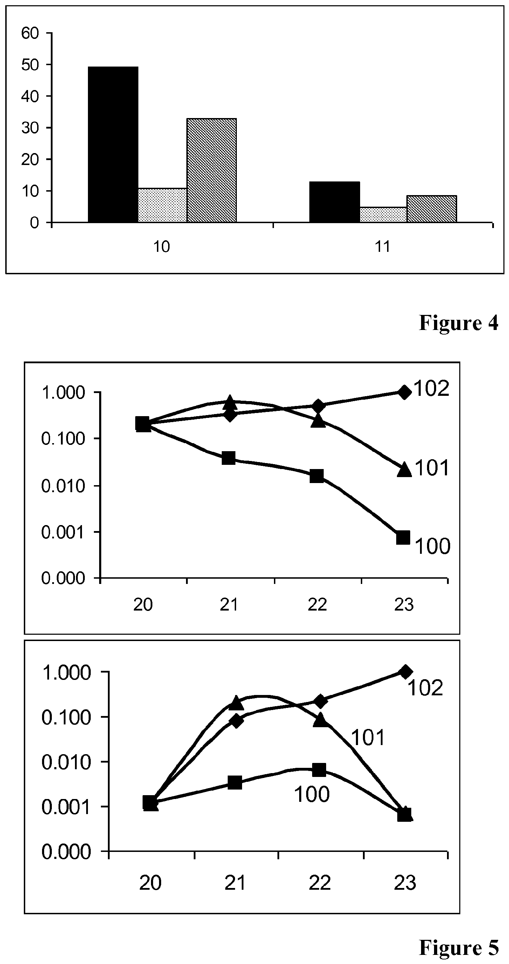

Figure 4 illustrates characterization of three tea brands using the present technology. Two different matrices were used.

Figure 5 illustrates determination of two water samples taken from two different lakes using three different matrices (same chelate and three different modulators in varying concentrations).

Figure 6 illustrates determination of four different tap water samples using six different matrixes (same metal, different ligands and modulators).

Figure 7 illustrates determination of water samples taken from four different rivers. Each river sample has characteristic kinetic curve.

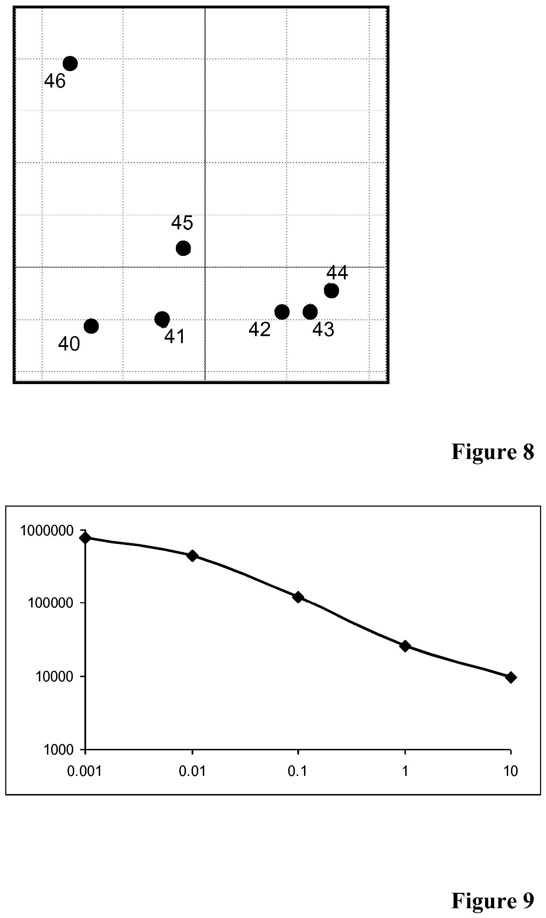

Figure 8 illustrates determination of seven different samples taken from different rivers (same metal, different ligands and modulators).

Figure 9 illustrates determination of samples containing different concentration of EDTA (same metal, same ligands and modulators).

Figure 10 illustrates determination of three tea samples. Europium(III) added together with the sample. Ligands are predissolved in DMSO.

Figure 11 illustrates determination of three household water samples. Eu : NTA:TOPO predissolved in a well (60). Eu predried in a separate spot than predissolved NTA:TOPO in DMSO. Sample only added.

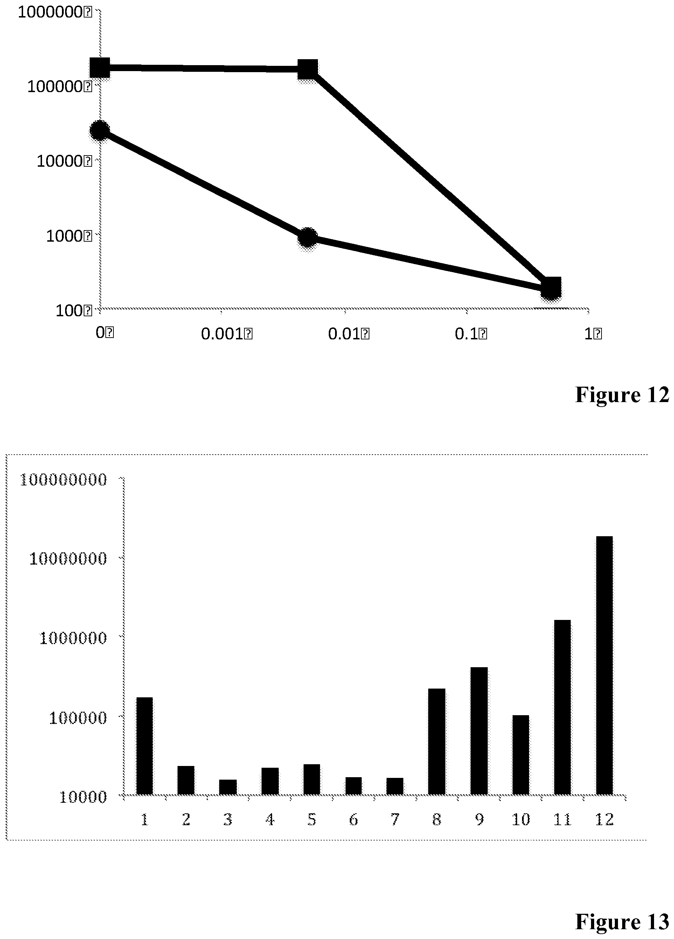

Figure 12 illustrates an example of characterization and/or determination of water samples containing different concentration of Fe³⁺. The matrix TbCl₃:dipicolinic acid and TbCl₃: 2,3-dihydroxynaphtalene were predried in a well. Sample only was added.

Figure 13 illustrates an example of different ligands. The matrix TbCl₃ and ligand was predried and water was added.

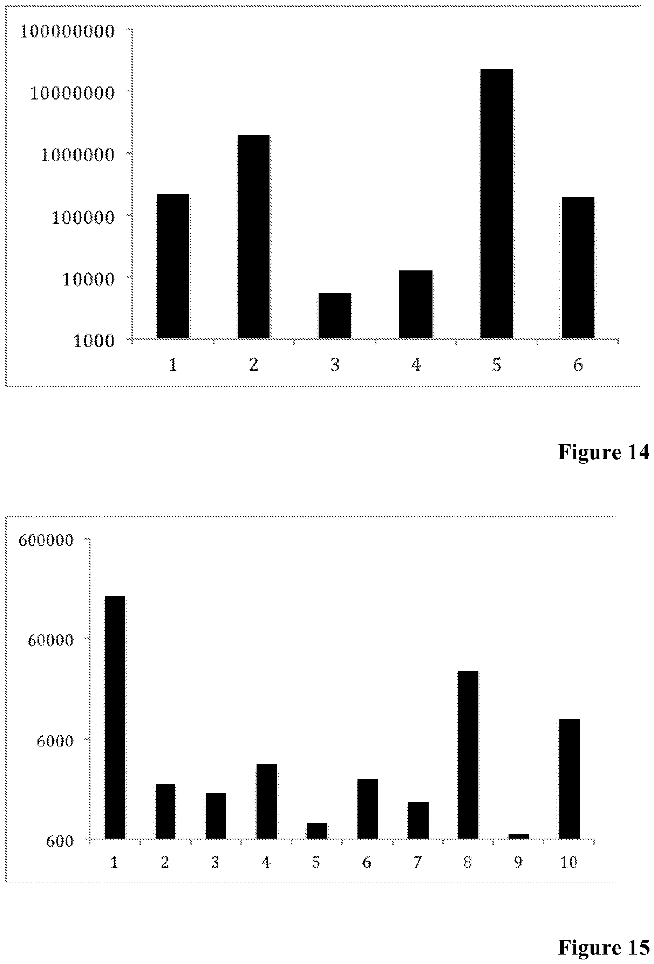

Figure 14 illustrates an example of different ligands. The matrix TbCl₃ and ligand was predried and water was added.

Figure 15 illustrates an example of measuring Fe³⁺. The matrix TbCl₃ and ligand was predried and water was added.

### EXAMPLES

Luminescence emission signal was measured with a 400-µs window after a 400-µs delay time using the Victor² multilabel counter (PerkinElmer, Turku, Finland) and 340-nm excitation and emission wavelengths of 615-nm for Eu and 545-nm for Tb.

### Example 1. Comparative analysis of tea samples

100 mg of Lipton yellow label (Unilever, UK), or Maryam broken (Intersun FZE, India), or Lipton blue fruit (Unilever, UK) tea were incubated with 1.0 mL of boiling deionized MilliQ water. After 10 min, the tea leaves were separated twice with centrifugation. The supernatant of the tea samples were diluted 10 000 -fold to MilliQ water and 100 µL were mixed with 2.0 µL of 34 µM of EuCl₃, 34 µM NTA and 102 µM TOPO (10) or 2.0 µL of 0.67 µM of EuCl₃, 18 µM NTA, 18 µM TOPO and 5 mM Triton X-100 (11) in MilliQ deionized in a 96-well microtiter plate. The luminescence signal of the solution was measured. The results are presented in Figure 4. Normalized luminescence signal is shown on the y-axis. Lipton yellow label (left), Maryam (middle), and Blue fruit (right) teas.

### Example 2: Comparative analysis of lake water samples in the presence of different concentrations of detergents

100 µL of lake water sample from Raisio and Eura was mixed with 2.0 µL of 3.4 µM of EuCl₃, 10 µM NTA and 10 µM TOPO EuCl₃ in deionized MilliQ water containing 0 (20), 5 (21) or 50 (22) mM SDS (100), 0 (20), 0.5 (21), 5 (22), or 50 (23) mM Tween20 (101) or 0 (20), 0.5 (21), 5 (22), or 50 (23) mM Triton X-100 (102) or 5.0 µL of 1.4 µM of EuCl₃, 4.2 µM NTA and 4.2 µM TOPO EuCl₃ in deionized MilliQ water containing 200 (23) mM SDS in a 96-well microtiter plate. The luminescence signal of the solution was measured. The results are presented in Figure 5. Normalized luminescence signal is shown on the y-axis.

### Example 3: Comparative analysis of household water samples in the presence of different ligands for europium(III) ion

100 µL of household water sample (Turku, Raisio, Pori, from top to down) was mixed with 2.0 µL of EuCl₃ in deionized MilliQ water and 2.0 µL of ligand or ligand mixture in deionized MilliQ water in a 96-well microtiter plate.. The final concentrations of EuCl₃ and ligands were: (30) 0.11 µM EuCl₃ and 1.2 mM trioctylphosphine oxide (TOPO); (31) 4.8 nM EuCl₃ and 29 µM 4,4,4-trifluoro-1-(2-naphthyl)-1,3-butanedione (NTA); (32) 4.8 nM EuCl₃, 29 µM NTA, and 29 µM TOPO; (33) 0.11 µM EuCl₃ and 1.2 mM dimethyldecylphosphine oxide (DMDPO); (34) 4.8 nM EuCl₃ and 29 µM thenoyltrifluoroacetone (TTA); or (35) 4.8 nM EuCl₃, 29 µM NTA, and 29 µM tributylphosphine oxide (TBBO). The luminescence signal of the solution was measured. The results are presented in Figure 6. Normalized luminescence signal is shown on the y-axis.

### Example 4: Comparative analysis of river water samples according to the changes in luminescence as a function of time

100 µL of river water samples (Eura (◆), Jaani (■), Aura (A), Raisio (×) river samples) were mixed with 2.0 µL of 34 µM of EuCl₃, 34 µM NTA and 102 µM TOPO (upper figure) or 2.0 µL of 0.67 µM of EuCl₃, 18 µM NTA, 18 µM TOPO in 100 mM sodium carbonate buffer pH 9.9 (lower figure) in a 96-well microtiter plate. The luminescence signal of the solution was measured at different time points: from left to right 0, 10, 23, 39, 57, 66, 81 min. The results are presented in Figure 7. Normalized luminescence signal is shown on the y-axis.

### Example 5: Comparative analysis of river water samples in the presence of different ligands for europium(III) ion

100 µL of river water sample (Aura (40), Raisio (41), Eura (42, 43), Köyliö (44), Kokemäki (45), or Jaani (46)) were mixed with 2.0 µL of EuCl₃ in deionized MilliQ water and 2.0 µL of ligand or ligand mixture in deionized MilliQ water in a 96-well microtiter plate. The final concentrations of EuCl₃ and ligands were: 0.11 µM EuCl₃ and 1.2 mM trioctylphosphine oxide (TOPO); 4.8 nM EuCl₃ and 29 µM 4,4,4-trifluoro-1-(2-naphthyl)-1,3-butanedione (NTA); 4.8 nM EuCl₃, 29 µM NTA, and 29 µM TOPO; 4.8 nM EuCl₃ and 29 µM 1,10-phenanthroline (phen); 4.8 nM EuCl₃, 29 µM phen, and 29 µM TOPO; 0.11 µM EuCl₃ and 1.2 mM triphenylphosphine oxide (TPPP); 4.8 nM EuCl₃, 29 µM NTA, and 29 µM triphenylphosphine oxide (TPPP); 0.11 µM EuCl₃ and 1.2 mM dimethyldecylphosphine oxide (DMDPO); 4.8 nM EuCl₃, 29 µM NTA, and 29 µM DMDPO; 4.8 nM EuCl₃ and 29 µM thenoyltrifluoroacetone (TTA); 4.8 nM EuCl₃, 29 µM TTA, and 29 µM TOPO; 0.11 µM EuCl₃ and 1.2 mM tributylphosphine oxide (TBBO); or 4.8 nM EuCl₃, 29 µM NTA, and 29 µM TBBO. The luminescence signal of the solution was measured. The two first principle components of the principle component analysis (Molegro) are presented in Figure 8.

### Example 6: Effect of concentration of EDTA on luminescence signal

100 µL of Raisio lake water sample were mixed with 2.0 µL of 3.4 µM of EuCl₃, 10 µM NTA and 10 µM TOPO in deionized MilliQ water containing 0, 0.01, 0.1, 1, or 10 mM EDTA in a 96-well microtiter plate. The luminescence signal of the solution was measured. The results are presented in Figure 9. The luminescence signal (y-axis) is shown as a function of EDTA (mM) concentration.

### Example 7: Comparative analysis of tea samples

3.0 µL of ligand or ligand mixture in DMSO was added to a 96-well micro titer plate. 100 µL of Lipton yellow label (left, Unilever, UK), Maryam broken (middle, Intersun FZE, India), and Lipton blue fruit (right, Unilever, UK) tea 1:3000-diluted in deionized MilliQ water was added together with 60 nM EuCl₃. The final concentrations of ligands were: (50) 0.17 µM 4,4,4-trifluoro-1-(2-naphthyl)-1,3-butanedione (NTA) and 0.17 µM trioctylphosphine oxide (TOPO); (51) 58 µM NTA; (52) 58 µM NTA and 58 µM dimethyldecylphosphine oxide (DMDPO); (53) 29 µM thenoyltrifluoroacetone (TTA) and 29 µM TOPO; and (54) 5.8 µM NTA and 5.8 µM 1,10-phenanthroline (phen). The luminescence signal was measured. The results are presented in Figure 10. Luminescence signal is shown on the y-axis.

### Example 8: Comparative analysis of household water samples

3.0 µL of solution containing EuCl₃, NTA, and TOPO in DMSO (60) or 3.0 µL of EuCl₃ in deionized MilliQ water was added to a 96-well microtiter plate and dried at 37 C and 3.0 µL of mixture of NTA and TOPO in DMSO was added to the opposite corner to a 96-well micro titer plate (61). 100 µL of Turku (left), Panelia (middle) or Pori (right) household water sample were added. The final concentrations of Eu³⁺, NTA, and TOPO were: 58 nM Eu³⁺, 0.17 µM NTA, and 0.17 µM TOPO (60) or 58 nM Eu³⁺, 1.7 µM NTA, and 1.7 µM TOPO (61). The luminescence signal was measured. The results are presented in Figure 11. Luminescence signal is shown on the y-axis.

### Example 9: Analysis of water samples containing Fe³⁺

3.0 µL of TbCl₃ and dipicolinic acid (square) or 2,3-dihydroxynaphtalene (circle) in deionized MilliQ water was added to a 96-well microtiter plate and dried at 37 C. 100 µL of water sample containing different concentration of Fe³⁺ were added. The final concentrations of Tb³⁺ and dipicolinic acid were: 50 µM Tb³⁺ and 1000 µM dipicolinic acid or 50 µM Tb³⁺ and 1000 µM 2,3-dihydroxynaphtalene. The luminescence signal was measured. The results are presented in Figure 12. Luminescence signal is shown on the y-axis and Fe³⁺ concentration (g/l) on the x-axis.

### Example 10: Comparison of different ligands

3 µL of TbCl₃, and a ligand in deionized MilliQ water was added to a 96-well microtiter plate and dried at 37 C. The ligands and TbCl₃ added were: dipicolinic acid (1), picolinic acid (2), nicotinic acid (3), 5-fluorosalicylic acid (4), 2,3-dihydroxynaphtalene (5), salicylic acid (6), cytosine (7), ciprofloxacin (11), nalidixic acid (12) at the final assay concentration of 50 µM Tb³⁺ and 1000 µM ligand, and chelidamic acid (8), tiron (9; sodium salt of 4,5-dihydrobenzene-1,3-disulfonic acid), oxolinic acid (10) at the final assay concentration of 5 µM Tb³⁺ and 50 µM ligand. 100 µL of 20 mM TRIS buffer were added. The luminescence signal was measured. The results are presented in Figure 13. Luminescence signal is shown on the y-axis and the ligand on the x-axis. It is clearly seen that the method using ligands (1) and (8)-(12) give rise to high signal. The method using especially ligands (8)-(10) give rise to high signal when the concentration of components is low

### Example 11: Comparison of different ligands

3 µL of TbCl₃, and a ligand in deionized MilliQ water was added to a 96-well microtiter plate and dried at 37 C. The ligands and TbCl₃ added were: dipicolinic acid (1), ciprofloxacin (2), 7-amino-1,3-naphtalenedisulfonic acid (3), 2,3-pyridinedicarboxylic acid (4), nalidixic acid (5), 4-hydroquinoline-2-carboxylic acid (6) at the final assay concentration of 50 µM Tb³⁺ and 1000 µM ligand. 100 µL of 20 mM TRIS buffer were added. The luminescence signal was measured. The results are presented in Figure 14. Luminescence signal is shown on the y-axis and the ligand on the x-axis.

### Example 12: Determination of Fe³⁺

3 µL of TbCl₃, and a ligand in deionized MilliQ water was added to a 96-well microtiter plate and dried at 37 C. The ligands and TbCl₃ added were: dipicolinic acid (1), picolinic acid (2), nicotinic acid (3), 5-fluorosalicylic acid (4), 2,3-dihydroxynaphtalene (5), salicylic acid (6), cytosine (7) at the final assay concentration of 50 µM Tb³⁺ and 1000 µM ligand, and chelidamic acid (8), tiron (9), oxolinic acid (10) at the final assay concentration of 5 µM Tb³⁺ and 50 µM ligand. 100 µL of deionized water were added. The luminescence signal was measured. The results are presented in Figure 15. Luminescence signal is shown on the y-axis and the ligand on the x-axis.

The specific examples provided in the description given above should not be construed as limiting the scope and/or applicability of the appended claims.

## Claims

1. A method for characterizing and/or determining a sample, the method comprising
a) providing an array comprising at least two different and separate matrices, each matrix including
- a ligand comprising an aromatic structure and 2-5 chelating heteroatoms, and
- optionally a lanthanide(III) ion,
b) introducing one or more solutions to said matrices wherein at least one of said solutions comprises
- said sample and
- optionally a lanthanide(III) ion;
*in proviso* that either said matrices or at least one of said solutions comprise said lanthanide(III) ion;
c) allowing said one or more solutions to dissolve said matrices;
d) detecting at a predetermined time point a signal derived from said lanthanide(III) ion in solution; and
e) characterizing and/or determining said sample by comparing said signal with
i) at least one signal derived from said lanthanide(III) ion in the presence of said dissolved matrices and one or more said solutions in the absence of said sample; and/or
ii) at least one signal derived from said lanthanide(III) ion in the presence of said dissolved matrices and one or more said solutions with corresponding sample; and/or
iii) at least one signal derived from said lanthanide(III) ion in the presence of said dissolved matrices and one or more said solutions with known sample,
wherein said ligand is selected from the group consisting of a compound of formula (II) 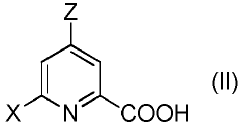
wherein Z is H or aryl, and X is selected from COOH and CH₂N(CH₂COOH)₂, and 1,10-phenantroline (phen), a compound of formula (III) 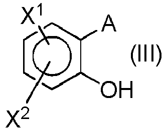
wherein A is OH or COOH, and X¹ and X² are independently selected from halogen, H, and SO₃H, and
a compound of formula (IV) 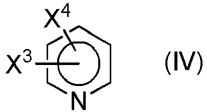
wherein X³ is selected from H, COOH and CONH₂, and X⁴ is selected from COOH and CONH₂.

2. The method according to claim 1 wherein the ligand is selected from the group consisting of dipicolinic acid, picolinic acid, nicotinic acid, nicotinamide, fluorosalisylic acid, 2,3-dihydronaphtalene, salicylc acid, cytosine, chelidamic acid, 4,5-dihydrobenzene-1,3-disulfonic acid, oxilinic acid, ciprofloxacin, 7-amino-1,3-naphhalenesulfonic acid, pyridinedicarboxylic acid, nalidixic acid, and 4-hydroquinoline-2-carboxylic acid.

3. The method according to any of claims 1-2 wherein said lanthanide is selected from europium, terbium, samarium and dysprosium, preferably europium.

4. The method of claim any of claims 1-3 wherein said matrices and/or said one or more solutions further comprise one or more modulators selected from the group consisting of synergistic ligand, detergent, protein, peptide, aminocarboxylic acid, carboxylic acid, buffer components, and carbohydrate.

5. The method of claim 4 wherein the synergistic ligand is selected from the group consisting of phosphine oxide preferably trioctylphosphine (TOPO), triphenylphosphine oxide (TPPO), dimethyldecylphosphine oxide (DMDPO), and tributylphosphine oxide (TBPO) and 1,10-phenantroline (phen).

6. The method of claim 4 or 5 wherein said detergent is selected from 4-(1,1,3,3-tetramethylbutyl)phenylpolyehyl glycol (Triton X-100), hexadecyltrimethylammonium bromide (CTAB), sodium docecyl sulfate (SDS), polyethyleneglycol sorbinan monolaurate (Tween-20) and dimethyldodecylphosphine oxide (Apo-12).

7. An array for characterizing and/or determining a sample, comprising at least two different and separate matrices, each matrix including a ligand comprising an aromatic structure and 2-5 chelating heteroatoms, and wherein said ligand is selected from a group consisting of a compound of formula (II) 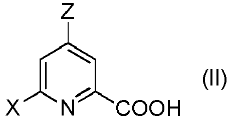
wherein Z is aryl, and X is selected from COOH and CH₂N(CH₂COOH)₂ and 1,10-phenantroline (phen),
a compound of formula (III) 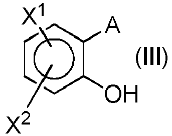
wherein A is OH or COOH, and X¹ and X² are independently selected from halogen, H, and SO₃H, and
a compound of formula (IV) 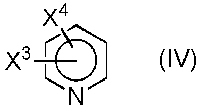
wherein X³ is selected from H, COOH and CONH₂, and X⁴ is selected from COOH and CONH₂.

8. The array according to claim 7, wherein the ligand is selected from the group consisting of dipicolinic acid, picolinic acid, nicotinic acid, nicotinamide, fluorosalicylic acid, 2,3-dihydronaphtalene, salicylcic acid, cytosine, chelidamic acid, 4,5-dihydrobenzene-1,3-disulfonic acid or its disodium salt, oxilinic acid, ciprofloxacin, 7-amino-1,3-naphhalenesulfonic acid, pyridinedicarboxylic acid, nalidixic acid, and 4-hydroquinoline-2-carboxylic acid.

9. The array according to claim 7 further comprising a lanthanide(III) ion selected from europium, terbium, samarium and dysprosium.

10. The array according any of claims 7-9 wherein said matrices further comprise one or more modulators selected from the group consisting of synergestic ligand, detergent, protein, peptide, aminocarboxylic acid, and carbohydrate.

11. The array according to claim 10 wherein the synergistic ligand is selected from the group consisting of phosphine oxide preferably trioctylphosphine (TOPO), triphenylphosphine oxide (TPPO), dimethyldecylphosphine oxide (DMDPO), and tributylphosphine oxide (TBPO) and 1,10-phenantroline (phen).

12. The array or claim 10 or 11 wherein said detergent is selected from 4-(1,1,3,3-tetramethylbutyl)phenylpolyehyl glycol (Triton X-100), sodium docecyl sulfate (SDS), Polyoxyethylene (20) sorbitan monolaurate (Tween-20), dimethyldodecylphosphine oxide (Apo-12), 3- [(3-cholamidopropyl) dimethylammonio]-1-propanesulfonate and cetyltrimethylammonium bromide (CTAB).

## Patentansprüche

1. Verfahren zum Charakterisieren und/oder Bestimmen einer Probe, wobei das Verfahren umfasst:
a) Bereitstellen einer Anordnung, die mindestens zwei unterschiedliche und getrennte Matrizen umfasst, wobei jede Matrix umfasst:
- einen Liganden, der eine aromatische Struktur und 2-5 chelatbildende Heteroatome umfasst und
- gegebenenfalls ein Lanthanoid(III)-Ion,
b) Einbringen einer oder mehrerer Lösungen in die Matrizen, wobei mindestens eine der Lösungen umfasst:
- die Probe und
- gegebenenfalls ein Lanthanoid(III)-Ion;
mit der Maßgabe, dass entweder die Matrizen oder mindestens eine der Lösungen das Lanthanoid(III)-Ion umfasst;
c) Ermöglichen, dass eine oder mehrere Lösungen die Matrizen auflösen;
d) Detektieren eines Signals, das von dem Lanthanoid(III)-Ion abgeleitet ist, zu einem vorbestimmten Zeitpunkt in Lösung; und
e) Charakterisieren und/oder Bestimmen der Probe durch Vergleichen des Signals mit
i) mindestens einem Signal, das von dem Lanthanoid(III)-Ion abgeleitet ist, in Gegenwart der gelösten Matrizen und einer oder mehreren Lösungen in Abwesenheit der Probe; und/oder
ii) mindestens einem Signal, das von dem Lanthanoid(III)-Ion abgeleitet ist, in Gegenwart der gelösten Matrizen und einer oder mehreren Lösungen mit entsprechender Probe; und/oder
iii) mindestens einem Signal, das von dem Lanthanoid(III)-Ion abgeleitet ist, in Gegenwart der gelösten Matrizen und einer oder mehreren Lösungen mit bekannter Probe,
wobei der Ligand ausgewählt ist aus der Gruppe bestehend aus einer Verbindung der Formel (II) 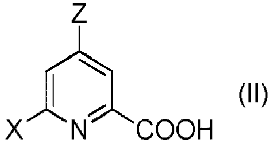
wobei Z H oder Aryl ist und X ausgewählt ist aus COOH und CH₂N(CH₂COOH)₂ und 1,10-Phenantrolin (Phen),
einer Verbindung der Formel (III) 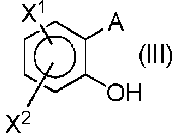
wobei A OH oder COOH ist und X¹ und X² unabhängig ausgewählt sind aus Halogen, H und SO₃H, und
einer Verbindung der Formel (IV) 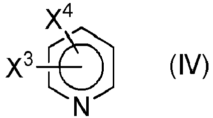
wobei X³ ausgewählt ist aus H, COOH und CONH₂ und X⁴ ausgewählt ist aus COOH und CONH₂.

2. Verfahren nach Anspruch 1, wobei der Ligand ausgewählt ist aus der Gruppe bestehend aus Dipicolinsäure, Picolinsäure, Nicotinsäure, Nicotinamid, Fluorsalicylsäure, 2,3-Dihydronaphtalin, Salicylsäure, Cytosin, Chelidamsäure, 4,5-Dihydrobenzol-1,3-disulfonsäure, Oxilinsäure, Ciprofloxacin, 7-Amino-1,3-naphhalinsulfonsäure, Pyridindicarbonsäure, Nalidixinsäure und 4-Hydrochinolin-2-carbonsäure.

3. Verfahren nach einem der Ansprüche 1-2, wobei das Lanthanoid ausgewählt ist aus Europium, Terbium, Samarium und Dysprosium, vorzugsweise Europium.

4. Verfahren nach einem der Ansprüche 1-3, wobei die Matrizen und/oder die eine oder die mehreren Lösungen ferner einen oder mehrere Modulatoren umfassen, die ausgewählt sind aus der Gruppe bestehend aus synergistischem Ligand, Detergens, Protein, Peptid, Aminocarbonsäure, Carbonsäure, Pufferkomponenten und Kohlenhydrat.

5. Verfahren nach Anspruch 4, wobei der synergistische Ligand ausgewählt ist aus der Gruppe bestehend aus Phosphinoxid, vorzugsweise Trioctylphosphin (TOPO), Triphenylphosphinoxid (TPPO), Dimethyldecylphosphinoxid (DMDPO) und Tributylphosphinoxid (TBPO) und 1,10-Phenantrolin (Phen).

6. Verfahren nach Anspruch 4 oder 5, wobei das Detergens ausgewählt ist aus 4-(1,1,3,3-Tetramethylbutyl)phenylpolyehylglycol (Triton X-100), Hexadecyltrimethylammoniumbromid (CTAB), Natriumdocecylsulfat (SDS), Polyethylenglycolsorbitanmonolaurat (Tween-20) und Dimethyldodecylphosphinoxid (Apo-12).

7. Anordnung zum Charakterisieren und/oder Bestimmen einer Probe, umfassend mindestens zwei unterschiedliche und getrennte Matrizen, wobei jede Matrix einen Liganden enthält, der eine aromatische Struktur und 2-5 chelatbildende Heteroatome umfasst, und wobei der Ligand ausgewählt ist aus einer Gruppe bestehend aus
einer Verbindung der Formel (II) 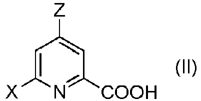
wobei Z Aryl ist und X ausgewählt ist aus COOH und CH₂N(CH₂COOH)₂ und 1,10-Phenantrolin (Phen),
einer Verbindung der Formel (III) 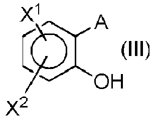
wobei A OH oder COOH ist und X¹ und X² unabhängig ausgewählt sind aus Halogen, H und SO₃H, und
einer Verbindung der Formel (IV) 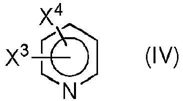
wobei X³ ausgewählt ist aus H, COOH und CONH₂ und X⁴ ausgewählt ist aus COOH und CONH₂.

8. Anordnung nach Anspruch 7, wobei der Ligand ausgewählt ist aus der Gruppe bestehend aus Dipicolinsäure, Picolinsäure, Nicotinsäure, Nicotinamid, Fluorsalicylsäure, 2,3-Dihydronaphtalin, Salicylsäure, Cytosin, Chelidamsäure, 4,5-Dihydrobenzol-1,3-disulfonsäure oder ihrem Dinatriumsalz, Oxilinsäure, Ciprofloxacin, 7-Amino-1,3-naphhalinsulfonsäure, Pyridindicarbonsäure, Nalidixinsäure und 4-Hydrochinolin-2-carbonsäure.

9. Anordnung nach Anspruch 7, ferner umfassend ein Lanthanoid(III)-Ion, das ausgewählt aus Europium, Terbium, Samarium und Dysprosium.

10. Anordnung nach einem der Ansprüche 7-9, wobei die Matrizen ferner einen oder mehrere Modulatoren umfassen, die ausgewählt sind aus der Gruppe bestehend aus synergistischem Ligand, Detergens, Protein, Peptid, Aminocarbonsäure und Kohlenhydrat.

11. Anordnung nach Anspruch 10, wobei der synergistische Ligand ausgewählt ist aus der Gruppe bestehend aus Phosphinoxid, vorzugsweise Trioctylphosphin (TOPO), Triphenylphosphinoxid (TPPO), Dimethyldecylphosphinoxid (DMDPO) und Tributylphosphinoxid (TBPO) und 1,10-Phenantrolin (phen).

12. Anordnung nach Anspruch 10 oder 11, wobei das Detergens ausgewählt ist aus 4-(1,1,3,3-Tetramethylbutyl)phenylpolyehylglycol (Triton X-100), Natriumdocecylsulfat (SDS), Polyoxyethylen(20)sorbitanmonolaurat (Tween-20), Dimethyldodecylphosphinoxid (Apo-12), 3-[(3-Cholamidopropyl)dimethylammonio]-1-propansulfonat und Cetyltrimethylammoniumbromid (CTAB).

## Revendications

1. Procédé pour la caractérisation et/ou la détermination d'un échantillon, le procédé comprenant
a) la fourniture d'un réseau comprenant au moins deux matrices différentes et distinctes, chaque matrice comprenant
- un ligand comprenant une structure aromatique et 2 à 5 hétéroatomes chélatants, et
- éventuellement un ion de lanthanide(III),
b) l'introduction d'une ou plusieurs solutions dans lesdites matrices, au moins l'une desdites solution comprenant
- ledit échantillon et
- éventuellement un ion de lanthanide(III),
à condition que l'une ou l'autres desdites matrices ou au moins l'une desdites solutions comprenne ledit ion de lanthanide(III) ;
c) le fait de laisser ladite ou lesdites solutions dissoudre lesdites matrices ;
d) la détection à un moment prédéterminé d'un signal issu dudit ion de lanthanide(III) en solution ; et
e) la caractérisation et/ou la détermination dudit échantillon par comparaison dudit signal avec
i) au moins un signal issu dudit ion de lanthanide(III) en la présence desdites matrices dissoutes et d'une ou plusieurs desdites solutions en l'absence dudit échantillon ; et/ou
ii) au moins un signal issu dudit ion de lanthanide(III) en la présence desdites matrices dissoutes et d'une ou plusieurs desdites solutions avec un échantillon correspondant ; et/ou
iii) au moins un signal issu dudit ion de lanthanide(III) en la présence desdites matrices dissoutes et d'une ou plusieurs desdites solutions avec un échantillon connu,
ledit ligand étant choisi dans le groupe constitué par un composé de formule (II) 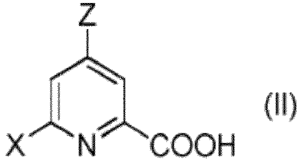
Z étant H ou aryle, et X étant choisi parmi COOH et CH₂N(CH₂COOH)₂, et la 1,10-phénanthroline (phen), un composé de formule (III) 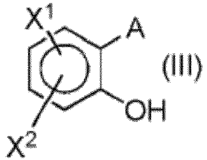
A étant OH ou COOH, et X¹ et X² étant indépendamment choisis parmi halogène, H, et SO₃H, et
un composé de formule (IV) 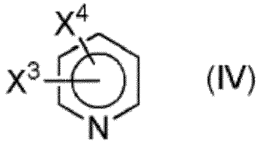
X³ étant choisi parmi H, COOH et CONH₂, et X⁴ étant choisi parmi COOH et CONH₂.

2. Procédé selon la revendication 1, le ligand étant choisi dans le groupe constitué par l'acide dipicolinique, l'acide picolinique, l'acide nicotinique, le nicotinamide, l'acide fluorosalisylique, le 2,3-dihydronaphtalène, l'acide salicylique, la cytosine, l'acide chélidamique, l'acide 4,5-dihydrobenzène-1,3-disulfonique, l'acide oxilinique, la ciprofloxacine, l'acide 7-amino-1,3-naphhalènesulfonique, l'acide pyridinedicarboxylique, l'acide nalidixique et l'acide 4-hydroquinoléine-2-carboxylique.

3. Procédé selon l'une quelconque des revendications 1 et 2, ledit lanthanide étant choisi parmi l'europium, le terbium, le samarium et le dysprosium, préférablement l'europium.

4. Procédé selon l'une quelconque des revendications 1 à 3, lesdites matrices et/ou ladite ou lesdites solutions comprenant en outre un ou plusieurs modulateurs choisis dans le groupe constitué par un ligand synergiste, un détergent, une protéine, un peptide, un acide aminocarboxylique, un acide carboxylique, des composants tampons, et un glucide.

5. Procédé selon la revendication 4, le ligand synergiste étant choisi dans le groupe constitué par un oxyde de phosphine, préférablement la trioctylphosphine (TOPO), l'oxyde de triphénylphosphine (TPPO), l'oxyde de diméthyldécylphosphine (DMDPO), et l'oxyde de tributylphosphine (TBPO) et la 1,10-phénanthroline (phen).

6. Procédé selon la revendication 4 ou 5, ledit détergent étant choisi parmi le 4-(1,1,3,3-tétraméthylbutyl)phénylpolyéthylèneglycol (Triton X-100), le bromure d'hexadécyltriméthylammonium (CTAB), le dodécylsulfate de sodium (SDS), le monolaurate de polyéthylèneglycol sorbitane (Tween-20) et l'oxyde de diméthyldodécylphosphine (Apo-12) .

7. Réseau pour la caractérisation et/ou la détermination d'un échantillon, comprenant au moins deux matrices différentes et distinctes, chaque matrice comprenant un ligand comprenant une structure aromatique et 2 à 5 hétéroatomes chélatants, et ledit ligand étant choisi dans un groupe constitué par un composé de formule (II) 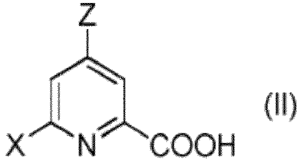
Z étant H ou aryle, et X étant choisi parmi COOH et CH₂N(CH₂COOH)₂, et la 1,10-phénanthroline (phen),
un composé de formule (III) 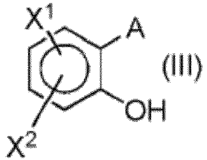
A étant OH ou COOH, et X¹ et X² étant indépendamment choisis parmi halogène, H, et SO₂H, et
un composé de formule (IV) 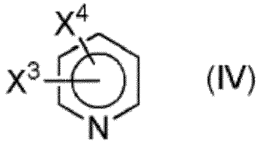
X³ étant choisi parmi H, COOH et CONH₂, et X⁴ étant choisi parmi COOH et CONH₂.

8. Réseau selon la revendication 7, le ligand étant choisi dans le groupe constitué par l'acide dipicolinique, l'acide picolinique, l'acide nicotinique, le nicotinamide, l'acide fluorosalisylique, le 2,3-dihydronaphtalène, l'acide salicylique, la cytosine, l'acide chélidamique, l'acide 4,5-dihydrobenzène-1,3-disulfonique ou son sel disodique, l'acide oxilinique, la ciprofloxacine, l'acide 7-amino-1,3-naphhalènesulfonique, l'acide pyridinedicarboxylique, l'acide nalidixique et l'acide 4-hydroquinoléine-2-carboxylique.

9. Réseau selon la revendication 7 comprenant en outre un ion de lanthanide(III) choisi parmi l'europium, le terbium, le samarium et le dysprosium.

10. Réseau selon l'une quelconque des revendications 7 à 9, lesdites matrices comprenant en outre un ou plusieurs modulateurs choisis dans le groupe constitué par un ligand synergiste, un détergent, une protéine, un peptide, un acide aminocarboxylique, et un glucide.

11. Réseau selon la revendication 10, le ligand synergiste étant choisi dans le groupe constitué par un oxyde de phosphine, préférablement la trioctylphosphine (TOPO), l'oxyde de triphénylphosphine (TPPO), l'oxyde de diméthyldécylphosphine (DMDPO), et l'oxyde de tributylphosphine (TBPO) et la 1,10-phénanthroline (phen).

12. Réseau selon la revendication 10 ou 11, ledit détergent étant choisi parmi le 4-(1,1,3,3-tétraméthylbutyl)phénylpolyéthylèneglycol (Triton X-100), le dodécylsulfate de sodium (SDS), le monolaurate de polyoxyéthylène (20) sorbitane (Tween-20), l'oxyde de diméthyldodécylphosphine (Apo-12), le 3-[(3-cholamidopropyl)diméthylammonio]-1-propanesulfonate et le bromure de cétyltriméthylammonium (CTAB).
